# EUROPEAN PATENT APPLICATION

(11) **EP 4 644 547 A1**
(43) Date of publication of application: **05.11.2025**
(21) Application number: 24775140.7
(22) Date of filing: 15.03.2024
(51) Int. Cl.: C12N 15/113, C12N 9/14, C12N 15/77, C12P 13/08

(54) **5'-UTR VARIANT SEQUENCES AND USE THEREOF**

(30) Priority: 17.03.2023 KR 20230035245
(71) Applicant: CJ CheilJedang Corporation, Seoul 04560 (KR)
(72) Inventor: KIM, Hye Mi, Seoul 04560 (KR); LEE, Hanhyoung, Seoul 04560 (KR); CHOI, Woosung, Seoul 04560 (KR)
(74) Representative: Meissner Bolte Partnerschaft mbB
(86) International application number: PCT/KR2024/003305
(87) International publication number: WO 2024/196092

(57) **Abstract**

The present disclosure relates to a 5'UTR variant sequence of *atpB* and a use of the same.

## Description

### [Technical Field]

The present disclosure relates to a 5'-UTR variant sequence, a microorganism comprising the same, and a method for producing L-lysine using the same.

### [Background Art]

A variety of studies have been conducted to develop microorganisms that produce L-amino acids with high efficiency and fermentation process technologies for the production of L-amino acids and other useful substances. For example, target substance-specific approaches such as increasing the expression of genes encoding enzymes involved in L-lysine biosynthesis or removing genes unnecessary for biosynthesis are mainly used (WO2008-082181 A1).

However, as the demand for L-lysine increases, studies to effectively increase the L-lysine production ability are still needed.

### [Disclosure]

### [Technical Problem]

The present disclosure relates to a 5'-UTR variant sequence, a microorganism comprising the same, and a method for producing L-lysine using the same.

### [Technical Solution]

An object of the present disclosure is to provide a variant polynucleotide encoding a variant 5'-untranslated region (5'-UTR) in which bases at positions corresponding to bases at 10th to 13th in a base sequence of SEQ ID NO: 13 are substituted with TCGG.

Another object of the present disclosure is to provide a variant polynucleotide comprising a base sequence encoding F₀F₁-ATP hydrolase (ATPase) or a subunit thereof, which further comprises a base sequence encoding a variant 5'-UTR in which bases at positions corresponding to bases at 10th to 13th in a base sequence of SEQ ID NO: 13 are substituted with TCGG.

Still another object of the present disclosure is to provide a microorganism that produces L-lysine, comprising a variant polynucleotide encoding a variant 5'-untranslated region (5'-UTR) in which bases at positions corresponding to bases at 10th to 13th in a base sequence of SEQ ID NO: 13 are substituted with TCGG; or a variant polynucleotide comprising a base sequence encoding F₀F₁-ATP hydrolase (ATPase) or a subunit thereof and further a base sequence encoding the variant 5'-UTR.

Still another object of the present disclosure is to provide a method for producing L-lysine, comprising culturing a microorganism in a medium wherein the microorganism comprises a variant polynucleotide encoding a variant 5'-untranslated region (5'-UTR) in which bases at positions corresponding to bases at 10th to 13th in a base sequence of SEQ ID NO: 13 are substituted with TCGG; or a variant polynucleotide comprising a base sequence encoding F₀F₁-ATP hydrolase (ATPase) or a subunit thereof and further a base sequence encoding the variant 5'-UTR, in a medium.

### [Advantageous Effects]

It is possible to increase L-lysine production ability by controlling the gene expression level using the 5'-untranslated region (5'-UTR) variant sequence of the present disclosure.

### [Detailed Description of Preferred Embodiments]

The present disclosure is explained in detail as follows. Meanwhile, each description and each embodiment disclosed in the present disclosure may also be applied to other descriptions and other embodiments, respectively. In other words, all combinations of various elements disclosed in the present disclosure fall within the scope of the present disclosure. Additionally, the scope of the present disclosure is not limited to the specific description described below.

Additionally, those skilled in the art will recognize, or be able to ascertain using no more than routine experimentation, a number of equivalents to the specific aspect of the present disclosure described herein. In addition, such equivalents are intended to be included in the present disclosure.

Additionally, a number of papers and patent documents are referenced and citations are indicated throughout this specification. The disclosures of the cited papers and patent documents are incorporated by reference into this specification in their entirety to more clearly explain the content of the present disclosure and the level of technical field to which the present disclosure belongs.

An aspect of the present disclosure is a variant polynucleotide encoding a variant 5'-untranslated region (5'-UTR) in which bases at positions corresponding to bases at 10th to 13th in a base sequence of SEQ ID NO: 13 are substituted with TCGG.

In the present disclosure, the term "5'-untranslated region (5'-UTR)" refers to a region that exists at the 5'-end of an mRNA transcript but is not translated into amino acids. The process of translating an mRNA into a protein begins with the binding of the 30S subunit of the ribosome to the 5'-UTR. Specifically, translation into a protein begins when 16S rRNA (16S ribosomal RNA) in the 30S subunit of the ribosome binds to an RBS in the 5'UTR, and tRNA recognizes and binds to the start codon (AUG) of the mRNA. It is known that the ribosome binding site of the 5'-UTR and the start codon are approximately 6 to 8 nucleotides apart.

In an embodiment, the 5'-UTR of the present disclosure may comprise a ribosome binding site (RBS).

In the present disclosure, the term "ribosome binding site (RBS)" refers to a region (structure), which is present inside the mRNA chain, to which a ribosome can directly bind, and at which translation can be initiated, or a region (structure) of a DNA chain, which creates this region by being transcribed. Most prokaryotes have RBS as a short sequence that makes it easier for a ribosome to recognize and bind to mRNA near the 5' upstream side from the start codon (usually 'AUG') on the mRNA comprising an open reading frame (ORF). In RBS, there is a sequence complementary to the sequence at the 3' end of 16S rRNA, which is called the Shine-Dalgarno sequence.

In any one of the preceding embodiments, the 5'-UTR may comprise a sequence in which a sequence complementary to the sequence at the 3' end of 16S rRNA, that is, the Shine-Dalgarno sequence is conserved and sequences before and after the Shine-Dalgarno sequence are regulated.

"Regulated 5'-UTR sequence" or "regulated 5'-UTR" means that one or more bases in the base sequence constituting the 5'UTR are modified compared to the wild-type sequence, and such modification may comprise substitution, addition, deletion, or inversion of bases.

As an example, the 5'-UTR may comprise, but is not limited to, sequences in which the Shine-Dalgarno sequence is conserved in the 5'-UTR and one or more bases in the base sequences present before and after the Shine-Dalgarno sequence are modified by substitution, addition, deletion, or inversion.

The 5'-UTR, which is the parent sequence of the variant 5'-UTR of the present disclosure, may be a sequence comprised in the 5'-UTR of a gene encoding F₀F₁-ATP hydrolase (ATPase) or a subunit thereof.

Accordingly, another aspect of the present disclosure is a variant polynucleotide comprising a base sequence encoding F₀F₁-ATP hydrolase (ATPase) or a subunit thereof, which further comprises a base sequence encoding a variant 5'-UTR in which bases at positions corresponding to bases at 10th to 13th in a base sequence of SEQ ID NO: 13 are substituted with TCGG.

In an embodiment, the 5'-UTR, which is the parent sequence of the variant 5'-UTR of the present disclosure, may comprise, have, or consist essentially of the base sequence of SEQ ID NO: 13.

In any one of the preceding embodiments, the 5'-UTR of the gene encoding F₀F₁-ATP hydrolase (ATPase) or a subunit thereof may comprise a base sequence having at least 70%, 75%, 76%, 80%, 84%, 85%, 88%, 90%, 92%, 95%, 96%, 97%, 98%, or 99% or more homology or identity to SEQ ID NO: 13 or the base sequence set forth by SEQ ID NO: 13. In addition, it is obvious that base sequences in which some sequences are deleted, modified, substituted, conservatively substituted, or added are also comprised within the scope of the present disclosure as long as they are base sequences that have such homology or identity and exhibit the same efficacy as that of the 5'-UTR sequence of SEQ ID NO: 13.

Meanwhile, SEQ ID NO: 1 of the present disclosure is a sequence comprising a portion of the sequence encoding atpB, a subunit of F₀F₁-ATP hydrolase (ATPase) and its 5'-UTR, and bases at 556th to 558th of SEQ ID NO: 1 correspond to the start codon. Bases at 1st to 555th of SEQ ID NO: 1 correspond to the 5'-UTR sequence and comprise SEQ ID NO: 13.

In any one of the preceding embodiments, the 5'-UTR sequence of the gene encoding F₀F₁-ATP hydrolase (ATPase) or a subunit thereof of the present disclosure may be the base sequence of SEQ ID NO: 1, comprising SEQ ID NO: 13.

In any one of the preceding embodiments, for the 5'-UTR sequence of the gene encoding F₀F₁-ATP hydrolase (ATPase) or a subunit thereof of the present disclosure may include a base sequence having at least 70%, 75%, 76%, 80%, 84%, 85%, 88%, 90%, 92%, 95%, 96%, 97%, 98%, or 99% or more homology or identity to SEQ ID NO: 1 or the base sequence set forth by SEQ ID NO: 1.

The variant 5'-UTR of the present disclosure comprises a variation in the ribosome binding site in the 5'-UTR. Therefore, the variant 5'-UTR of the present disclosure may also be referred to as a "variant RBS-comprising sequence."

The variant 5'-UTR of the present disclosure may be a 5'-UTR sequence comprising substitution of the base corresponding to 10th base of the base sequence of SEQ ID NO: 13 with T, substitution of the base corresponding to 11th base of the base sequence of SEQ ID NO: 13 with C, substitution of the base corresponding to 12th base of the base sequence of SEQ ID NO: 13 with G, and substitution of the base corresponding to 13th base of the base sequence of SEQ ID NO: 13 with G, that is, substitution of the bases at the positions corresponding to bases at 10th to 13th with TCGG.

In an embodiment, the variant 5'-UTR of the present disclosure may comprise, have, or consist essentially of the base sequence of SEQ ID NO: 14.

In any one of the preceding embodiments, the variant 5'-UTR of the present disclosure may have, comprise, or consist essentially of a base sequence in which the bases at the positions corresponding to bases at 10th to 13th of SEQ ID NO: 13 are fixed to TCGG and the base sequence is at least 70% or more, 75% or more, 76% or more, 80% or more, 81 % or more, 82% or more, or 83% or more of the entire sequence compared to SEQ ID NO: 13, but is not limited thereto.

In addition, it is obvious that a variant 5'UTR having a base sequence in which some sequences are deleted, modified, substituted, conservatively substituted, or added is also comprised within the scope of the present disclosure as long as it is a base sequence that has such homology or identity and exhibits efficacy corresponding to that of the variant 5'UTR of the present disclosure.

In the present disclosure, the term "polynucleotide" is a polymer of nucleotides in which nucleotide monomers are linked to each other in a long chain shape by covalent bonds, and refers to a DNA or RNA strand with a certain length or more.

In the present disclosure, the term "variant" refers to a polynucleotide in which one or more bases are modified and the base sequence of the variant polynucleotide is different from the base sequence before variation but the functions or properties are maintained. Such a variant can generally be identified by modifying one or more bases in the base sequence of the polynucleotide and evaluating the properties of the modified polynucleotide. In other words, the ability of the variant polynucleotide may be increased, unchanged, or decreased compared to that of the polynucleotide before being varied. The variant polynucleotide may be used interchangeably with terms such as variant, modification, modified polynucleotide, modified gene, mutant, mutein, and divergent, and is not limited thereto as long as it is used in a sense of variation.

The variant polynucleotide may be conjugated with other sequences or linkers so as to be identified, purified, or synthesized.

In the present disclosure, the term "corresponding to" refers to a base at the position listed in a polynucleotide, or to a base that is similar, identical, or homologous to the base listed in a polynucleotide. Identifying the base at the corresponding position may be determining the specific base in a sequence that references a specific sequence. As used herein, "corresponding region" generally refers to a similar or corresponding position in a related polynucleotide sequence or a reference polynucleotide sequence.

For example, an arbitrary base sequence may be aligned with SEQ ID NO: 13 or SEQ ID NO: 1, and based on this, each base in the base sequence may be numbered by referring to SEQ ID NO: 13 or SEQ ID NO: 1 and the numerical position of a base corresponding to the base. For example, a sequence alignment algorithm such as that described in the present disclosure can identify the position of a base or the position of a modification such as a substitution, insertion, or deletion by comparison to a query sequence (also referred to as a "reference sequence").

For this alignment, for example, the Needleman-Wunsch algorithm (Needleman and Wunsch, 1970, J. Mol. Biol. 48: 443-453), the Needleman program of the EMBOSS package (EMBOSS: The European Molecular Biology Open Software Suite, Rice et al., 2000), Trends Genet. 16: 276-277), and the like may be used, but the algorithm program is not limited thereto, and sequence alignment programs, pairwise sequence comparison algorithms, and the like known in the art may be appropriately used.

In the present disclosure, the term 'homology' or 'identity' means the degree of similarity between two given amino acid sequences or base sequences and may be expressed as a percentage. The terms homology and identity may often be used interchangeably.

The sequence homology or identity of conserved polynucleotides or polypeptides is determined by standard alignment algorithms, and default gap penalties established by the program being used may be used together. Substantially homologous or identical sequences are generally capable of hybridizing with each other in all or part of the sequences under moderate or high stringent conditions. It is obvious that hybridization also includes hybridization with a polynucleotide containing a common codon or a codon considering the codon degeneracy in polynucleotides.

Whether any two polynucleotide or polypeptide sequences have homology, similarity, or identity may be determined, for example, using known computer algorithms such as the "FASTA" program using default parameters as in Pearson et al (1988) [Proc. Natl. Acad. Sci. USA 85]: 2444. Alternatively, the homology, similarity, or identity may be determined using the Needleman-Wunsch algorithm (Needleman and Wunsch, 1970, J. Mol. Biol. 48: 443-453) as performed in the Needleman program of the EMBOSS package (EMBOSS: The European Molecular Biology Open Software Suite, Rice et al., 2000, Trends Genet. 16: 276-277) (version 5.0.0 or later) (including the GCG program package (Devereux, J., et al, Nucleic Acids Research 12: 387 (1984)), BLASTP, BLASTN, FASTA (Atschul, [S.] [F.,] [ET AL, J MOLEC BIOL 215]: 403 (1990); Guide to Huge Computers, Martin J. Bishop, [ED.,] Academic Press, San Diego,1994, and [CARILLO ETA/.](1988) SIAM J Applied Math 48: 1073). For example, BLAST of the National Center for Biotechnology Information Database, or ClustalW may be used to determine homology, similarity, or identity.

The homology, similarity or identity of polynucleotides or peptides may be determined by comparing sequence information using, for example, a GAP computer program such as Needleman et al. (1970), J Mol Biol. 48:443, as noted in, for example, Smith and Waterman, Adv. Appl. Math (1981) 2:482. In summary, using the GAP program, the homology, similarity, or identity may be defined by the value obtained by dividing the number of similarly aligned symbols (namely, nucleotides or amino acids) by the total number of symbols in the shorter of two sequences. The default parameters for the GAP program include (1) a unitary matrix (containing values of 1 for identity and 0 for non-identity) and a weighted comparison matrix of Gribskov et al (1986) Nucl. Acids Res. 14: 6745 (or EDNAFULL (EMBOSS version of NCBI NUC4.4) substitution matrix) as disclosed by Schwartz and Dayhoff, eds., Atlas Of Protein Sequence And Structure, National Biomedical Research Foundation, pp. 353-358 (1979); (2) a penalty of 3.0 for each gap and an additional 0.10 penalty for each symbol in each gap (or 10 gap opening penalty, 0.5 gap extension penalty); and (3) no penalty for end gaps.

The polynucleotide of the present disclosure may undergo various modifications in the coding region within a range of not changing the base sequence of a variant gene comprising the variant 5'-UTR of the present disclosure considering the codon degeneracy or the preferred codon in an organism seeking to express the variant gene comprising the variant 5'-UTR of the present disclosure.

In addition, the polynucleotide of the present disclosure may comprise a probe that can be prepared from a known genetic sequence, for example, a sequence that can hybridize under stringent conditions with a sequence complementary to all or part of the polynucleotide sequence of the present disclosure without limitation. The "stringent condition" refers to conditions that enable specific hybridization between polynucleotides. These conditions are specifically described in the literatures (see J. Sambrook et al., Molecular Cloning, A Laboratory Manual, 2nd Edition, Cold Spring Harbor Laboratory press, Cold Spring Harbor, New York, 1989; F.M. Ausubel et al., Current Protocols in Molecular Biology, John Wiley & Sons, Inc., New York, 9.50-9.51, 11.7-11.8). Examples thereof include conditions in which polynucleotides having high homology or identity, namely, polynucleotides having 70% or more, 75% or more, 76% or more, 80% or more, 84% or more, 85% or more, 88% or more, 90% or more, 92% or more, 95% or more, 96% or more, 97% or more, 98% or more, 99% or more, 99.5% or more, 99.7% or more, 99.8% or more, 99,9% or more homology or identity hybridize with each other and polynucleotides having homology or identity lower than this do not hybridize with each other; or conditions in which washing is performed 1 time, specifically 2 to 3 times at a salt concentration and temperature corresponding to 60°C, 1XSSC, and 0.1% SDS, specifically 60°C, 0.1XSSC, and 0.1% SDS, more specifically 68°C, 0.1XSSC, and 0.1% SDS, which are washing conditions for conventional southern hybridization.

Hybridization requires that two nucleic acids have complementary sequences although mismatches between bases are possible depending on the stringency of hybridization. The term "complementary" is used to describe the relation between nucleotide bases capable of hybridizing with each other. For example, with regard to DNA, adenine is complementary to thymine and cytosine is complementary to guanine. Hence, the polynucleotide of the present disclosure may also comprise substantially similar nucleic acid sequences as well as isolated nucleic acid fragments complementary to the entire sequence.

Specifically, polynucleotides having homology or identity with the polynucleotide of the present disclosure may be detected under hybridization conditions comprising a hybridization step at a Tm value of 55°C and the conditions described above. The Tm value may be 60°C, 63°C or 65°C, but is not limited thereto, and may be appropriately adjusted by those skilled in the art depending on the purpose.

The appropriate stringency for hybridizing polynucleotides depends on the length and degree of complementarity of the polynucleotides, and the parameters are well known in the art (for example, Sambrook et al., supra).

The variant polynucleotide encoding the variant 5'-UTR of the present disclosure may regulate the translation of a polynucleotide encoding a target protein operably linked thereto into a protein. The target protein comprises proteins without limitation as long as the expression thereof can be regulated by the variant 5'-UTR of the present disclosure.

In an embodiment, the variant polynucleotide encoding the 5'-UTR may be comprised at the upstream of the base sequence encoding F₀F₁-ATP hydrolase (ATPase) or a subunit thereof.

In any one of the preceding embodiments, the variant polynucleotide encoding the variant 5'-UTR may be operably linked to the *atpBEFHAGDC* operon to regulate the activity of one or more of the subunit proteins (subunits) constituting a complex of F₀F₁-ATP hydrolase.

In any one of the preceding embodiments, the variant polynucleotide encoding the variant 5'-UTR may be operably linked to the *atpB* gene.

In any one of the preceding embodiments, the variant polynucleotide encoding the variant 5'-UTR may regulate the expression of the F₀F₁-ATP hydrolase operon.

In the present disclosure, the term "F₀F₁-ATP hydrolase (F₀F₁-ATPase)" is an enzyme that can synthesize ATP using the proton gradient (proton motive force) in the cell membrane, and functions to synthesize ATP in a general cellular environment and is thus also referred to as ATP synthase. It is known that F₀F₁-ATPase of microorganisms of the genus *Corynebacterium* comprises 8 subunits and is encoded by the *atpBEFHAGDC* operon comprising 8 genes.

In the present disclosure, "operon" means a functional unit of DNA comprising a gene group whose the expression is regulated by one expression regulatory sequence, specifically, one promoter. The mRNA transcribed by an operon may be a polycistronic mRNA, in which a single mRNA molecule codes for one or more proteins, or a monocistronic mRNA, in which a single mRNA molecule codes for one protein.

The operon of the present disclosure may comprise an expression regulatory region and a structure gene. The "expression regulatory region" of an operon refers to a region that exists upstream of a structure gene constituting the operon and can regulate the expression of the structure gene. The expression regulatory region of the operon may comprise the 5'-UTR region excluding the structure gene. The expression regulatory region may comprise an RBS region.

Still another aspect of the present disclosure is a vector comprising a variant polynucleotide encoding a variant 5'-untranslated region (5'-UTR) in which bases at positions corresponding to bases at 10th to 13th in a base sequence of SEQ ID NO: 13 are substituted with TCGG.

Still another aspect of the present disclosure is a vector comprising a variant polynucleotide comprising a base sequence encoding F₀F₁-ATP hydrolase (ATPase) or a subunit thereof, which further comprises a base sequence encoding a variant 5'-UTR in which bases at positions corresponding to bases at 10th to 13th in a base sequence of SEQ ID NO: 13 are substituted with TCGG.

The variant polynucleotide, base sequence of SEQ ID NO: 13, and variant 5'-UTR are as described above.

The vector may be an expression vector for expressing a polynucleotide in a host cell, but is not limited thereto.

The vector of the present disclosure may comprise a DNA product comprising the base sequence of a target polynucleotide operably linked to a suitable expression regulatory region (or expression regulatory sequence) so as to express the target polynucleotide in a suitable host. The expression regulatory region comprises a promoter capable of initiating transcription, an arbitrary operator sequence for regulating such transcription, a sequence encoding a suitable RBS, and a sequence regulating the termination of transcription and translation.

For the purposes of the present disclosure, the expression regulatory region may be the variant 5'-UTR described in the previous aspect. As an example, the target polynucleotide may be a base sequence encoding F₀F₁-ATP hydrolase or a subunit thereof. As an example, the target polynucleotide may be a base sequence encoding the α-subunit of the F₀ part of F₀F₁-ATP hydrolase. As an example, the target polynucleotide may be the *atpBEFHAGDC* operon. As an example, the target polynucleotide may be the *atpB* gene or NCgl1159. However, the target polynucleotide comprises polynucleotides without limitation as long as the expression thereof can be regulated by the variant 5'-UTR of the present disclosure.

After being transformed into a suitable host cell, the vector can replicate or function independently of the host genome and can be integrated into the genome itself.

The vector used in the present disclosure is not particularly limited, and an arbitrary vector known in the art may be used. Examples of commonly used vectors comprise plasmids, cosmids, viruses, and bacteriophages in a natural or recombinant state. For example, pWE15, M13, MBL3, MBL4, IXII, ASHII, APII, t10, t11, Charon4A, and Charon21A may be used as phage vectors or cosmid vectors, and pDZ-based, pBR-based, pUC-based, pBluescriptll-based, pGEM-based, pTZ-based, pCL-based and pET-based plasmid vectors may be used as plasmid vectors. Specifically, pDZ, pDC, pDCM2, pACYC177, pACYC184, pCL, pECCG117, pUC19, pBR322, pMW118, and pCC1BAC vectors, and the like may be used.

As an example, a target polynucleotide may be inserted into a chromosome using a vector for intracellular chromosome insertion. Insertion of a polynucleotide into a chromosome may be accomplished by an arbitrary method known in the art, for example, homologous recombination, but is not limited thereto. A selection marker to examine the insertion of a polynucleotide into a chromosome may be additionally comprised. The selection marker is used to select cells transformed with the vector, that is, to examine the insertion of a target nucleic acid molecule, and markers that confer a selectable phenotype, such as drug resistance, auxotrophy, resistance to cytotoxic agents, or expression of surface polypeptides, may be used. In an environment treated with a selective agent, only cells expressing the selection marker survive or exert other expression traits, so transformed cells can be selected.

In the present disclosure, the term "transformation" refers to introducing a vector comprising a target polynucleotide into a host cell or microorganism so that the polynucleotide can be expressed in the host cell. The transformed polynucleotide may be inserted into the chromosome of a host cell or located outside the chromosome as long as it can be expressed in the host cell. Additionally, the polynucleotide comprises DNA and/or RNA encoding a target polypeptide. The polynucleotide may be introduced in any form as long as it can be introduced into and expressed in a host cell. For example, the polynucleotide may be introduced into a host cell in the form of an expression cassette, which is a genetic structure comprising all elements necessary for self-expression. The expression cassette may typically comprise a promoter, a transcription termination signal, a ribosome binding site, and a translation termination signal that are operably linked to the polynucleotide. The expression cassette may be in the form of an expression vector capable of self-replication. In addition, the polynucleotide may be introduced into a host cell in its own form and operably linked to the sequence required for expression in the host cell, but is not limited thereto.

In addition, the term "operably linked" as used above means that the expression regulatory sequence comprising the base sequence encoding the variant 5'-UTR of the present disclosure and the base sequence encoding a target protein are functionally linked. As an example, the base sequence encoding a target protein may be a base sequence encoding F₀F₁-ATP hydrolase or a subunit thereof. As an example, the base sequence encoding a target protein may be the *atpBEFHAGDC* operon. As an example, the base sequence encoding a target protein may be a base sequence encoding the α-subunit of the F₀ part of F₀F₁-ATP hydrolase. As an example, the base sequence encoding a target protein may be the *atpB* gene or NCgl1159.

As an example, the variant 5'-UTR of the present disclosure can regulate the translation of a polynucleotide encoding a target protein operably linked thereto into a protein, but is not limited thereto.

Still another aspect of the present disclosure provides a microorganism that produces L-lysine, comprising a variant polynucleotide encoding a variant 5'-untranslated region (5'-UTR) in which bases at positions corresponding to bases at 10th to 13th in a base sequence of SEQ ID NO: 13 are substituted with TCGG; or a variant polynucleotide comprising a base sequence encoding F₀F₁-ATP hydrolase (ATPase) or a subunit thereof and further a base sequence encoding the variant 5'-UTR.

In an embodiment, the microorganism of the present disclosure may comprise a variant polynucleotide encoding a variant 5'-UTR in which the bases at the positions corresponding to bases at 10th to 13th in a base sequence of SEQ ID NO: 13 are substituted with TCGG and further a polynucleotide encoding F₀F₁-ATP hydrolase or a subunit thereof.

In any one of the preceding embodiments, the microorganism of the present disclosure may comprise a vector comprising the polynucleotide.

In any one of the preceding embodiments, the microorganism of the present disclosure may comprise any one or more selected from a variant polynucleotide encoding a variant 5'-untranslated region (5'-UTR) in which bases at positions corresponding to bases at 10th to 13th in a base sequence of SEQ ID NO: 13 are substituted with TCGG; a variant polynucleotide containing a base sequence encoding F₀F₁-ATP hydrolase (ATPase) or a subunit thereof and further a base sequence encoding the variant 5'-UTR; or a vector containing the polynucleotide.

In the microorganism of the present disclosure, 5'-UTR, F₀F₁-ATP hydrolase, variant polynucleotide, vector, and the like are as described in the other aspects.

In the present disclosure, the term "microorganism (or strain)" comprises both wild-type microorganisms and microorganisms that have undergone natural or artificial genetic modification, is a microorganism in which a specific mechanism is weakened or enhanced by causes such as insertion of exogenous genes or enhancement or inactivation of the activity of endogenous genes, and may be a microorganism that comprises genetic modification for expression of a target polynucleotide.

The microorganism may be a microorganism of the genus *Corynebacterium.*

As an example of the present disclosure, the microorganism of the present disclosure may be *Corynebacterium glutamicum, Corynebacterium crudilactis, Corynebacterium deserti, Corynebacterium efficiens, Corynebacterium callunae, Corynebacterium stationis, Corynebacterium singulare, Corynebacterium halotolerans, Corynebacterium striatum, Corynebacterium ammoniagenes, Corynebacterium pollutisoli, Corynebacterium imitans, Corynebacterium testudinoris* or *Corynebacterium flavescens.*

The microorganism of the present disclosure may be specifically a microorganism of the genus *Corynebacterium,* more specifically *Corynebacterium glutamicum,* but is not limited thereto.

The microorganism of the present disclosure may have the ability to produce L-lysine.

The microorganism of the present disclosure may be a natural wild-type microorganism, an unmodified microorganism, or a recombinant microorganism in which a variant polynucleotide that can be transcribed into the variant 5'-UTR of the present disclosure; or a variant polynucleotide encoding F₀F₁-ATP hydrolase or a subunit thereof, comprising the variant polynucleotide that can be transcribed into the variant 5'-UTR is introduced into a microorganism that produces L-lysine.

In the present disclosure, the term "unmodified microorganism" does not exclude strains comprising mutations that may occur naturally in microorganisms, and may mean a wild-type strain or natural strain itself, or a strain before its traits are changed by genetic variation due to natural or artificial factors. For example, the unmodified microorganism may mean a strain in which a variant polynucleotide encoding the variant 5'-UTR described herein; or a variant polynucleotide encoding F₀F₁-ATP hydrolase or a subunit thereof, comprising the variant polynucleotide that can be transcribed into the variant 5'-UTR is not introduced or is not yet introduced. As an example, the unmodified microorganism may mean a strain comprising the base sequence of SEQ ID NO: 1 or SEQ ID NO: 13. The "unmodified microorganism" may be used interchangeably with "strain before being modified", "microorganism before being modified", "unvaried strain", "unmodified strain", "unvaried microorganism" or "reference microorganism".

In an embodiment, in the microorganism of the present disclosure, the activity of proteins or genes involved in the L-lysine biosynthetic pathway can be further enhanced or weakened.

In any one of the preceding embodiments, the microorganism of the present disclosure may comprise pyc (P458S), hom (V59A) and lysC (T311I) variations (Binder et al. Genome Biology 2012, 13:R40; US 9593354 B2).

In any one of the preceding embodiments, in the microorganism of the present disclosure, additionally the activity of any one or more selected from aspartate kinase, transketolase, or pyruvate carboxylase may be enhanced.

In any one of the preceding embodiments, in the microorganism of the present disclosure, additionally the activity of any one or more selected from the lysC gene, tkt gene, or pyc gene may be enhanced. In any one of the preceding embodiments, in the microorganism of the present disclosure, the start codon of any one or more genes selected from the lysC gene, tkt gene, or pyc gene may be substituted with ATG.

Still another aspect of the present disclosure is a method for producing L-lysine, comprising culturing a microorganism in a medium wherein the microorganism comprises a variant polynucleotide encoding a variant 5'-untranslated region (5'-UTR) in which bases at positions corresponding to bases at 10th to 13th in a base sequence of SEQ ID NO: 13 are substituted with TCGG; or a variant polynucleotide comprising a base sequence encoding F₀F₁-ATP hydrolase (ATPase) or a subunit thereof and further a base sequence encoding the variant 5'-UTR, in a medium.

In the method of the present disclosure, 5'-UTR, F₀F₁-ATP hydrolase, variant polynucleotide, microorganism, and the like are as described in the other aspects.

In the present disclosure, the term "culture" means growing the microorganism of the present disclosure under appropriately controlled environmental conditions. The culture process of the present disclosure may be carried out using appropriate media and culture conditions known in the art. This culture process can be easily adjusted and used by those skilled in the art depending on the selected microorganism. Specifically, the culture may be batch, continuous, and/or fed-batch culture, but is not limited thereto.

In the present disclosure, the term "medium" refers to a material in which nutrients necessary for culturing the microorganism of the present disclosure are mixed as main components, and supplies nutrients and growth factors, comprising water, which are essential for survival and development. Specifically, as the medium and other culture conditions used for culturing the microorganism of the present disclosure, any medium used for culturing ordinary microorganisms may be used without any particular limitation. The microorganism of the present disclosure may be cultured in a normal medium containing appropriate carbon sources, nitrogen sources, phosphorus sources, inorganic compounds, amino acids, vitamins, and/or the like under aerobic conditions while temperature, pH, and the like are controlled.

In the present disclosure, the carbon sources may comprise carbohydrates such as glucose, saccharose, lactose, fructose, sucrose, and maltose; sugar alcohols such as mannitol and sorbitol, organic acids such as pyruvic acid, lactic acid, and citric acid; and amino acids such as glutamic acid, methionine, and lysine. Additionally, natural organic nutrient sources such as starch hydrolysate, molasses, blackstrap molasses, rice bran, cassava, bagasse and corn steep liquor may be used. Specifically, carbohydrates such as glucose and pre-sterilized molasses (namely, molasses converted to reducing sugars) may be used, and appropriate amounts of other carbon sources may be variously used without limitation. These carbon sources may be used singly or in combination of two or more thereof, but are not limited thereto.

As the nitrogen sources, inorganic nitrogen sources such as ammonia, ammonium sulfate, ammonium chloride, ammonium acetate, ammonium phosphate, ammonium carbonate, and ammonium nitrate; and organic nitrogen sources such as amino acids such as glutamic acid, methionine, and glutamine, peptone, NZ-amine, meat extract, yeast extract, malt extract, corn steep liquor, casein hydrolysate, fish or decomposition products thereof, and defatted soybean cake or decomposition products thereof may be used. These nitrogen sources may be used singly or in combination of two or more thereof, but are not limited thereto.

Examples of the phosphorus sources may include monopotassium phosphate and dipotassium phosphate or monosodium phosphate and disodium phosphate. As the inorganic compounds, sodium chloride, calcium chloride, iron chloride, magnesium sulfate, iron sulfate, manganese sulfate, calcium carbonate, and the like may be used, and amino acids, vitamins, appropriate precursors and/or the like may be used in addition to these. These components or precursors may be added to the medium either batchwise or continuously, but the manner to add the components or precursors is not limited thereto.

During the culture of the microorganism of the present disclosure, compounds such as ammonium hydroxide, potassium hydroxide, ammonia, phosphoric acid, and sulfuric acid may be added to the medium in an appropriate manner to adjust the pH of the medium. During culture, the formation of bubbles may be suppressed by using antifoaming agents such as fatty acid polyglycol esters. Oxygen or oxygen-containing gas may be injected into the medium in order to maintain the aerobic state of the medium, or gas may not be injected or nitrogen, hydrogen, or carbon dioxide gas may be injected in order to maintain anaerobic and microaerobic states, but the atmosphere controlling method is not limited thereto.

In the culture of the present disclosure, the culture temperature may be maintained at 20°C to 45°C, specifically 25°C to 40°C, and the culture time may be about 10 to 160 hours, but the culture conditions are not limited thereto.

L-lysine produced by the culture of the present disclosure may be secreted into the medium or remain in the cells.

The L-lysine production method of the present disclosure may further comprise preparing the microorganism of the present disclosure, preparing a medium for culturing the microorganism, or a combination thereof (in any order), for example, prior to the culture step.

The L-lysine production method of the present disclosure may further comprise recovering L-lysine from the medium used in culture (medium subjected to culture) or the cultured microorganism. The recovery step may be further comprised after the culture step.

The recovery may be to collect the desired L-lysine using a suitable method known in the art according to the method for culturing a microorganism of the present disclosure, for example, batch, continuous or fed-batch culture method. For example, centrifugation, filtration, treatment (salting out) with a precipitating agent for crystallized proteins, extraction, sonication, ultrafiltration, dialysis, various kinds of chromatography such as molecular sieve chromatography (gel filtration), adsorption chromatography, ion exchange chromatography, and affinity chromatography, HPLC, or a combination thereof may be used. The desired L-lysine may be recovered from the medium or microorganism by suitable methods known in the art.

The L-lysine production method of the present disclosure may further comprise a purification step. The purification may be performed by suitable methods known in the art. In an example, when the L-lysine production method of the present disclosure comprises both a recovery step and a purification step, the recovery step and the purification step may be performed continuously or discontinuously in any order, or may be performed simultaneously or by being integrated into one step, but the manner to perform the recovery step and the purification step is not limited thereto.

Still another aspect of the present disclosure provides a composition for L-lysine production comprising a microorganism that produces L-lysine, comprising a variant polynucleotide encoding a variant 5'-untranslated region (5'-UTR) in which bases at positions corresponding to bases at 10th to 13th in a base sequence of SEQ ID NO: 13 are substituted with TCGG or a variant polynucleotide comprising a base sequence encoding F₀F₁-ATP hydrolase (ATPase) or a subunit thereof and further a base sequence encoding the variant 5'-UTR; a medium in which the microorganism has been cultured; or a combination of two or more thereof.

The composition of the present disclosure may further comprise arbitrary suitable excipients commonly used in compositions for L-glutamine production, and such excipients may be, for example, preservatives, wetting agents, dispersing agents, suspending agents, buffers, stabilizers, or tonicity agents, but are not limited thereto.

Still another aspect of the present disclosure provides a use of a microorganism comprising a variant polynucleotide encoding a variant 5'-untranslated region (5'-UTR) in which bases at positions corresponding to bases at 10th to 13th in a base sequence of SEQ ID NO: 13 are substituted with TCGG; or a variant polynucleotide comprising a base sequence encoding F₀F₁-ATP hydrolase (ATPase) or a subunit thereof and further a base sequence encoding the variant 5'-UTR for L-lysine production.

The 5'-UTR, F₀F₁-ATP hydrolase, variant polynucleotide, microorganism and the like are as described in the other aspects.

### [Examples]

Hereinafter, the present disclosure will be described in more detail through Examples and Experimental Examples. However, these Examples and Experimental Examples are for illustrative purposes only and the scope of the present disclosure is not limited to these Examples and Experimental Examples.

### Example 1. Specification of atpB upstream sequence variant utilizing UTR Designer (RBS prediction program)

Three RBS sequence variants were designed to predict protein expression level and control the protein expression level, and prediction of the expression levels thereof was performed using UTR Designer (https://sbi.postech.ac.kr/utr designer/). In order to predict the current protein expression level of the target gene atpB, the upstream 25-nucleotide sequence of the *atpB* gene and the 35-nucleotide sequence comprising the start codon of the target gene were input and the 16s rRNA sequence [CCUCCUUUC] of *Corynebacterium* was input. The protein expression level of *atpB* predicted in the program was predicted to be about 6 (Table 1). The three designed variant sequences and expected expression levels thereof are listed in Table 2.

The RBS sequence variants were named atpB RBS1 (56) / atpB RBS2 (974) / atpB RBS3 (1802) in order from lowest protein expression intensity.

**[Table 1]**

| Start | Spacinge | Sequence | dG_{UTR} | Expression Level |
|---|---|---|---|---|
| 25 | 10 | AACGCTGAGCGTTACAACACTGTCCATGAAGG GCGAATTCCACGCCCCCGATTTGGACAA | 12.88 | 5.94 |

### [Expression level of wild-type atpB predicted by RBS prediction program]

**[Table 2]**

| | 5'-UTR Sequence | Protein Coding Sequence | dG_{UTR} | Expression Level |
|---|---|---|---|---|
| 1 | AACGCTGAGTCGGACAAC ACTGTCC | ATGAAGGGCGAATTCCACGCCCC CGATTTGGACAA | 8.85 | 56 |
| 2 | AACGCTGAGGAGGACAAC ACTGTCC | ATGAAGGGCGAATTCCACGCCCC CGATTTGGACAA | 3.76 | 974 |
| 3 | AACGCTGAGGAGGCCAAC ACTGTCC | ATGAAGGGCGAATTCCACGCCCC CGATTTGGACAA | 2.66 | 1802 |

### [Protein expression level for each sequence predicted through UTR Designer]

### Example 2. Construction of vector comprising predicted sequence and introduction of strain

In order to construct a vector comprising each predicted RBS sequence, primers were prepared based on SEQ ID NO: 1. In order to construct a vector comprising the atpB RBS1 sequence using primer SEQ ID NOs: 2 and 3 and primer SEQ ID NOs: 4 and 5 in pairs, template PCR was performed using the chromosome of *Corynebacterium glutamicum* ATCC13032 as a template. Additionally, in order to construct a vector comprising the atpB RBS2/3 sequence, template PCR was performed using each pair of primer SEQ ID NOs: 2 and 6, primer SEQ ID NOs: 7 and 5; primer SEQ ID NOs: 2 and 8, and primer SEQ ID NOs: 9 and 5, respectively. As the PCR condition, after denaturation at 94°C for 5 minutes, PCR was repeated 30 times at 94°C for 30 seconds, 55°C for 30 seconds, and 72°C for 1 minute and 30 seconds, and then performed at 72°C for 5 minutes. Each template PCR fragment was subjected to fusion cloning after the plasmid pDCM2 (Korea Publication No. 10-2020-0136813) was treated with restriction enzyme Smal for insertion and replacement of genes in the *Corynebacterium* chromosome. The In-Fusion^{®} **HD** cloning kit (Clontech) was used, and the template PCR fragment was then transformed into E. *coli* DH5α and plated on an **LB** solid medium comprising kanamycin (25 mg/l). Colonies transformed with the vector into which the target gene was inserted were selected through PCR using primer SEQ **ID** NOs: 10 and 11, then plasmids were obtained using a commonly known plasmid extraction method, and the plasmids were named pDC_atpB RBS1, pDC_atpB RBS2, and pDC_atpB RBS3, respectively. The sequences of the primers used in the present Example are listed in Table 3 below.

**[Table 3]**

| Primer SEQ ID NO: | Sequence (5' -> 3') |
|---|---|
| SEQ ID NO: 2 | AGAACGCTGAGTCGGACAACACTGTCCATGAAGGGCGAAT |
| SEQ ID NO: 3 | CTCTAGAGGATCCCC CAAACGCAGTGCCAGGGTGACTGGC |
| SEQ ID NO: 4 | GACAGTGTTGTACTCCTTAGCGTTCTCTCCCGTGTTCGGG |
| SEQ ID NO: 5 | AGAACGCTAAGGAGTACAACACTGTCCATGAAGGGCGAAT |
| SEQ ID NO: 6 | TGTTGTCCTCCTCAGCGTTCTCTCCCGTGTTCGGGCCGTG |
| SEQ ID NO: 7 | GAGAACGCTGAGGAGGACAACACTGTCCATGAAGGGCGAA |
| SEQ ID NO: 8 | AGTGTTGGCCTCCTCAGCGTTCTCTCCCGTGTTCGGGCCG |
| SEQ ID NO: 9 | AGAGAACGCTGAGGAGGCCAACACTGTCCATGAAGGGCGA |
| SEQ ID NO: 10 | ATCGCCATTCGCCATTCAGG |
| SEQ ID NO: 11 | TCATTAATGCAGCTGGCACG |

The vectors pDC_atpB RBS1, pDC_atpB RBS2, pDC_atpB RBS3, and pDC_atpB RBS4 constructed in Example 1 were transformed into *Corynebacterium glutamicum* CJ3P (US 9556463 B2) by the electric pulse method (Van der Rest et al., Appl. Microbiol. Biotecnol. 52:541-545, 1999). Strains in which variation was caused upstream of the *atpB* gene by homologous chromosomal recombination were identified using primer pairs with sequences of SEQ ID NOs: 2 and 5, and named CJ3P_atpB-RBS1, CJ3P_atpB-RBS2, and CJ3P_atpB-RBS3, respectively.

### Example 3. Comparison of L-lysine production ability of constructed atpB RBS variant strains

Flask fermentation titer evaluation was performed to examine the L-lysine production ability of the strains constructed in Example 2 and the control parent strain.

First, each strain was inoculated into a 250 ml corner-baffle flask containing 25 ml of seed medium, and cultured at 37°C for 20 hours with shaking at 200 rpm. Into a 250 ml corner-baffle flask containing 24 ml of production medium, 1 ml of seed culture solution was inoculated, and cultured at 37°C for 36 hours with shaking at 200 rpm. In order to accurately measure the time of glycogen consumption, 700 µL of culture solution was taken at the time of 18 hours of culture, the L-lysine concentration was measured, and the production ability of each strain was calculated. The production ability (g/L/h), rate of increase in production ability (%), and lysine concentration (g/L) after completion of culture for each strain tested are as presented in Table 4 below.

The experiment was repeated three times, and the average values of the analysis results thereof are presented in Table 4 below.

### <Seed medium (pH 7.0)>

Glucose 20 g, peptone 10 g, yeast extract 5 g, urea 1.5 g, KH₂PO₄ 4 g, K₂HPO₄ 8 g, MgSO₄ 7H₂O 0.5 g, biotin 0.1 mg, thiamine HCl 1 mg, calcium-pantothenic acid 2 mg, and nicotinamide 2 mg (based on 1 liter of distilled water)

### <Production medium (pH 7.0)>

Glucose 100 g, (NH₄)₂SO₄ 40 g, soybean protein 2.5 g, corn steep solids 5 g, urea 3 g, KH₂PO₄ 1 g, MgSO₄ 7H₂O 0.5 g, biotin 100 µg, thiamine hydrochloride 1000 µg, calcium pantothenate 2000 µg, nicotinamide 3000 µg, and CaCO₃ 30 g (based on 1 liter of distilled water).

**[Table 4]**

| Strain | Lysine concentration (g/L) | | Lysine productivity (g/L/h) | Degree of improvement in lysine productivity (%) |
|---|---|---|---|---|
| | 18h | 36h | at 18h | |
| CJ3P | 4.25 | 7.94 | 0.177 | 100 |
| CJ3P_atpB-RBS1 | 4.41 | 8.01 | 0.184 | 103.8 |
| CJ3P_atpB-RBS2 | 3.89 | 7.59 | 0.162 | 91.5 |
| CJ3P_atpB-RBS3 | 0.23 | 0.33 | 0.009 | 5.4 |

As presented in Table 4, it was verified that among the three atpB RBS variants, only the CJ3P_atpB-RBS1 strain, which comprised the atpB RBS1 variant, had L-lysine production ability increased to about 103.8% compared to that of the control CJ3P. The results showed that the other variants rather resulted in decreased lysine concentrations and had decreased lysine production ability. In particular, in the case of the CJ3P_atpB-RBS3 strain, it was verified that the growth of the strain was completely inhibited. Through these results, it has been verified that *atpB* expression regulated by the specific sequence of atpB RBS1 is required to improve lysine production ability.

Based on the above description, it will be understood by those skilled in the art that the present disclosure may be implemented in a different specific form without changing the technical spirit or essential characteristics thereof. Therefore, it should be understood that the above embodiment is not limitative, but illustrative in all aspects. The scope of the disclosure is defined by the appended claims rather than by the description preceding them, and therefore all changes or modified forms derived from the meaning and scope of the claims and their equivalent concepts should be construed as being included in the scope of the present disclosure.

## Claims

1. A variant polynucleotide encoding a variant 5'-untranslated region (5'-UTR) in which bases at positions corresponding to bases at 10th to 13th in a base sequence of SEQ ID NO: 13 are substituted with TCGG.

2. A variant polynucleotide comprising a base sequence encoding F₀F₁-ATP hydrolase (ATPase) or a subunit thereof, wherein
the variant polynucleotide further comprises a base sequence encoding a variant 5'-UTR in which bases at positions corresponding to bases at 10th to 13th in a base sequence of SEQ ID NO: 13 are substituted with TCGG.

3. The variant polynucleotide according to claim 1 or 2, wherein the variant 5'-UTR comprises a base sequence of SEQ ID NO: 14.

4. The variant polynucleotide according to claim 1, wherein the variant polynucleotide encoding the variant 5'-UTR is operably linked to a polynucleotide encoding a target protein.

5. The variant polynucleotide according to claim 1, wherein the variant polynucleotide encoding the variant 5'-UTR is operably linked to an *atpB* gene.

6. The variant polynucleotide according to claim 1, wherein the variant polynucleotide encoding the variant 5'-UTR regulates expression of an F₀F₁-ATP hydrolase operon.

7. The variant polynucleotide according to claim 1 or 2, wherein the base sequence of SEQ ID NO: 13 comprises a ribosome binding site (RBS).

8. A microorganism that produces L-lysine, comprising:
a variant polynucleotide encoding a variant 5'-untranslated region (5'-UTR) in which bases at positions corresponding to bases at 10th to 13th in a base sequence of SEQ ID NO: 13 are substituted with TCGG; or
a variant polynucleotide comprising a base sequence encoding F₀F₁-ATP hydrolase (ATPase) or a subunit thereof and further a base sequence encoding the variant 5'-UTR.

9. A method for producing L-lysine, comprising
culturing a microorganism in a medium
wherein the microorganism comprises a variant polynucleotide encoding a variant 5'-untranslated region (5'-UTR) in which bases at positions corresponding to bases at 10th to 13th in a base sequence of SEQ ID NO: 13 are substituted with TCGG; or a variant polynucleotide comprising a base sequence encoding F₀F₁-ATP hydrolase (ATPase) or a subunit thereof and further a base sequence encoding the variant 5'-UTR.

10. The method according to claim 9, wherein the method further comprises recovering L-lysine from the cultured microorganism, the culture of the microorganism, the fermentation of the microorganism, or the culture medium.
